# EUROPÄISCHE PATENTANMELDUNG

(11) **EP 4 748 402 A1**
(43) Veröffentlichungstag der Anmeldung: **27.05.2026**
(21) Anmeldenummer: 24214806.2
(22) Anmeldetag: 22.11.2024
(51) Int. Cl.: A61L 2/20, A61L 2/28

(54) **VERFAHREN ZUR ERMITTLUNG EINER WIRKSAMKEITSAUSSAGE IN EINEM DEKONTAMINATIONSPROZESS UND KORRESPONDIERENDE KONTROLLIERTE UMGEBUNG**

(71) Anmelder: SKAN AG, 4123 Allschwil (CH)
(72) Erfinder: Lehmann, Frank, 4102 Binningen (CH); Sommerhalder, Matthias, 4112 Bättwil (CH); Kemmerling, Dr. Simon, 4106 Therwil (CH); Krebsbach, Dr. Timo, 4310 Rheinfelden (CH); Novák, Martin, 4102 Binningen (CH); Eggert, Benjamin, 02763 Zittau (DE); Hommes, Dr. Gregor, 47669 Wachtendonk (DE)
(74) Vertreter: Mertzlufft-Paufler, Cornelius

(57) **Zusammenfassung**

Es wird ein Verfahren zur Ermittlung einer Wirksamkeitsaussage in einem Dekontaminationsprozess (1) vorgeschlagen, wobei in dem Dekontaminationsprozess (1) ein Dekontaminationsmittel (2) auf wenigstens ein Objekt (4) in einer kontrollierte Umgebung (3) einwirkt, wobei eine mit einer Konzentration des Dekontaminationsmittels (2) in der kontrollierten Umgebung (3) korrelierende Konzentrations-Messgröße (7), eine mit einem Feuchtigkeitsgehalt in der kontrollierten Umgebung korrelierende Feuchte-Messgröße (8) und eine mit einer Temperatur in der kontrollierten Umgebung korrelierende Temperatur-Messgröße (9) erfasst werden so dass mit den erfassten Messgrößen (7, 8, 9) die Wirksamkeitsaussage, vorzugsweise automatisiert, ermittelt wird.

## Beschreibung

Die Erfindung betrifft ein Verfahren zur Ermittlung einer Wirksamkeitsaussage in einem Dekontaminationsprozess, wobei in dem Dekontaminationsprozess ein Dekontaminationsmittel in eine kontrollierte Umgebung zu einer Einwirkung auf wenigstens ein Objekt eingebracht wird.

Die Erfindung betrifft weiter ein Verfahren zur Bewertung eines Dekontaminationsprozesses, der in einer kontrollierten Umgebung ausgeführt wird.

Kontrollierte Umgebungen sind bekannt und können beispielsweise dadurch charakterisierbar sein, dass Zustandsparameter, beispielsweise Druck, Temperatur, Luftzusammensetzung, Luftströmungsgeschwindigkeit und/oder Luftfeuchte, und/oder ein Stoffaustausch der Umgebung mit deren Außenwelt kontrolliert definierbar ist/sind. Eine Liste von Beispielen für kontrollierte Umgebungen umfassen Containments, insbesondere Isolatoren und Gloveboxes, und Restricted Access Barrier Systems (Zugangsbeschränktes Barrieresystem, RABS), insbesondere vom offenen oder geschlossenen Typ. Kontrollierte Umgebungen werden beispielsweise eingesetzt, um während eines vorzugsweise industriellen Prozesses eine unerwünschte Wechselwirkung mit der Außenwelt zu vermindern oder zu eliminieren. Ein beispielhafter Anwendungsfall kann das Abfüllen oder Umpacken eines Medikaments sein, ein anderer eine sterile Montage eines Applikators für eine Medikament.

Zur Vorbereitung der kontrollierten Umgebung wird häufig ein Dekontaminationsprozess durchgeführt, durch welchen vermehrungsfähige Verunreinigungen biologisch deaktiviert werden. Hierzu wirkt ein Dekontaminationsmittel auf die kontrollierte Umgebung ein. Als Dekontaminationsmittel sind beispielsweise lebensfeindliche Substanzen wie Wasserstoffperoxid oder lebensfeindliche und/oder ionisierende Strahlungen wie UV- und/oder Gamma-Strahlung und/oder hochenergetische Teilchen-Strahlung verwendbar.

Der Erfindung liegt die Aufgabe zugrunde, den Dekontaminationsprozess in kontrollierten Umgebungen zu verbessern.

Zur Lösung der genannten Aufgabe sind erfindungsgemäß die Merkmale von Anspruch 1 vorgesehen. Insbesondere wird somit zur Lösung der genannten Aufgabe erfindungsgemäß vorgeschlagen, dass eine mit einer Konzentration des Dekontaminationsmittels in der kontrollierten Umgebung korrelierende Konzentrations-Messgröße, eine mit einem Feuchtigkeitsgehalt in der kontrollierten Umgebung korrelierende Feuchte-Messgröße und eine mit einer Temperatur in der kontrollierten Umgebung korrelierende Temperatur-Messgröße erfasst werden und dass mit den erfassten Messgrößen die Wirksamkeitsaussage ermittelt wird. Hierdurch kann beispielsweise erreichbar sein, dass der Dekontaminationsprozess verbessert wird, da erstmals die Konzentration des Dekontaminationsmittels, beispielsweise Wasserstoffperoxid (H2O2), gemessen werden kann, um den Dekontaminationsprozess anzusteuern. Somit kann beispielsweise die Zufuhr des Dekontaminationsmittels in Abhängigkeit von der Konzentration des Dekontaminationsmittels in der kontrollierten Umgebung gesteuert werden. Vorzugsweise kann die Wirksamkeitsaussage automatisiert ermittelt werden. Hierdurch kann beispielsweise auf die Ressource Mensch verzichtet werden, um schneller und kostensparender zu arbeiten. Unnötig lange Dekontaminationsprozesse sind vermeidbar.

Allgemein kann eine Wirksamkeitsaussage beispielsweise dadurch charakterisierbar sein, dass sie eine Aussage über einen Grad einer Durchführung einer Dekontamination ermöglicht. Als ein Beispiel für eine Wirksamkeitsaussage kann der kill factor angesehen werden, also die Aussage, um welchen Faktor oder um welche Zehnerpotenz biologisch vermehrungsfähiges Material durch die Dekontamination vermindert wird. Die Ermittlung, insbesondere Berechnung, einer Wirksamkeitsaussage hat den Vorteil, dass der betrachtete Prozess vergleichbar mit bekannten Prozessen und insbesondere überwachbar wird, also ob er mit etablierten Überprüfungsverfahren wie zum Beispiel solchen, bei denen chemische oder Bio-Indikatoren eingesetzt werden, überwacht wird. Die Erfindung hat hier den Vorteil, dass ein Ergebnis der Überwachung sofort und insbesondere ohne langwierige Inkubation oder sonstige biologische Vermehrung bereitsteht.

Bei einer vorteilhaften Ausgestaltung kann vorgesehen sein, dass für die Ermittlung der Wirksamkeitsaussage zusätzlich eine Zeitdauer einer Einwirkung des Dekontaminationsmittels verarbeitet wird. Hierdurch kann beispielsweise eine Mindestzeitspanne für die Dekontamination geschaffen werden. Auch kann durch eine Zeiterfassung ein Daten-Logging der zuvor beschriebenen Messgrößen erfolgen.

Bei einer vorteilhaften Ausgestaltung kann vorgesehen sein, dass für die Ermittlung der Wirksamkeitsaussage zusätzlich eine Strömungsgeschwindigkeit in der kontrollierten Umgebung verarbeitet wird. Somit ist eine besonders genaue und/oder aussagekräftige Wirksamkeitsaussage erzeugbar.

Bei einer vorteilhaften Ausgestaltung kann vorgesehen sein, dass als Dekontaminationsmittel Wasserstoffperoxid verwendet wird. Wasserstoffperoxid kann vermehrungsfähige Verunreinigungen biologisch deaktivieren und ein Wachstum solcher Verunreinigungen damit verhindern. Vorzugsweise kann in einer Gasphase aufgenommenes Wasserstoffperoxid verwendet werden. Hierdurch kann beispielsweise erreichbar sein, dass das Wasserstoffperoxid nicht vorab verdampft werden muss. Als Gasphase kann das Wasserstoffperoxid alle zu dekontaminierenden Flächen in der kontrollierten Umgebung benetzen.

Bei einer vorteilhaften Ausgestaltung kann vorgesehen sein, dass wenigstens zwei Messgrößen an einem gemeinsamen Messort in der kontrollierten Umgebung erfasst werden. Hierdurch kann beispielsweise ein Zusammenhang zwischen den Messgrößen hergestellt werden, der einen Rückschluss auf die Wirksamkeit des Dekontaminationsprozesses zulässt. Insbesondere kann vorgesehen sein, dass die drei Messgrößen an einem gemeinsamen Messort in der kontrollierten Umgebung erfasst werden. Hierdurch kann beispielsweise ein Zusammenhang zwischen den drei Messgrößen hergestellt werden, der einen Rückschluss auf die Wirksamkeit des Dekontaminationsprozesses zulässt.

Bei einer vorteilhaften Ausgestaltung kann vorgesehen sein, dass als Wirksamkeitsaussage eine Kennzahl, die mit einem Anteil von den die Kontamination überstehenden Mikroorganismen korreliert, berechnet wird. Hierdurch kann einem Anwender beispielsweise schnell und einfach die Wirksamkeit der Dekontamination vermittelt werden.

Bei einer vorteilhaften Ausgestaltung kann vorgesehen sein, dass die Ermittelung der Wirksamkeitsaussage in einem Kalibriermodus anhand von wenigstens einem aufgestellten Bioindikator und/oder wenigstens einem chemischen Indikator eingelernt und/oder validiert wird. Somit ist eine gute Vergleichbarkeit mit bisherigen Validierungsverfahren erreichbar.

Bei einer vorteilhaften Ausgestaltung kann vorgesehen sein, dass eine relative Sättigung und/oder ein Taupunkt für das Dekontaminationsmittel bestimmt wird. Somit lassen sich besonders gut definierte Bedingungen für eine wirkungsvolle Dekontamination erreichen.

Beispielsweise kann die Bestimmung dadurch erreicht werden, dass Werte für eine Steuerung vorgegeben und/oder Werte gemessen werden. Durch eine Vorgabe für die Steuerung können prozessgünstige Werte angefahren werden. Durch eine Messung ist ein Regelkreis zur Ansteuerung von hinterlegten Sollwerten einrichtbar.

Insbesondere kann hierbei vorgesehen sein, dass in der kontrollierten Umgebung eine relative Sättigung von wenigstens 80% oder wenigstens 95%, insbesondere 100%, erreicht wird. Versuche haben ergeben, dass bei derart hohen Sättigungen die Unterschiede im Mess- und/oder Ansprechverhalten zwischen verschiedenen Bioindikatoren verschwinden oder zumindest vernachlässigbar sind.

Alternativ oder zusätzlich sind zur Lösung der eingangs genannten Aufgabe erfindungsgemäß die Merkmale des nebengeordneten, auf ein Verfahren zur Bewertung des Dekontaminationsprozesses gerichteten Anspruchs vorgesehen. Insbesondere wird somit vorgeschlagen, dass in einem Einrichtungsmodus wenigstens ein Referenzpunkt und ein Netz von mehreren Messpunkten in der kontrollierten Umgebung definiert wird, wobei während einer ersten Durchführung des Dekontaminationsprozesses Messwerte an dem wenigstens einen Referenzpunkt und an den Messpunkten aufgenommen werden und wobei ein funktioneller Zusammenhang zwischen den Messwerten des wenigstens einen Referenzpunktes und den Messwerten der Messpunkte ermittelt wird, und dass in einem Betriebsmodus wenigstens ein Messwert des wenigstens einen Referenzpunktes aufgenommen wird und dass in dem Betriebsmodus aus dem wenigstens einen aufgenommenen Messwert mit dem funktionalen Zusammenhang wenigstens eine auf wenigstens einen Messpunkt des Netzes bezogene Information automatisiert ermittelt wird.

Hierdurch kann beispielsweise erreichbar sein, dass eine Bewertung des Dekontaminationsprozesses möglich ist, bei dem von dem wenigstens einen Referenzpunkt auf die gesamte kontrollierte Umgebung geschlossen werden kann. Somit kann in dem Betriebsmodus auf die mehreren Messpunkte verzichtet werden, was Kosten sparen kann. Es sind auch Messungen an kritischen, aber schwer zugänglichen Stellen einfach ersetzbar durch Messungen an einfach zugänglichen Stellen.

Bei einer vorteilhaften Ausgestaltung kann vorgesehen sein, dass an jedem Referenzpunkt wenigstens eine mit einer Konzentration des Dekontaminationsmittels in der kontrollierten Umgebung korrelierende Konzentrations-Messgröße, eine mit einem Feuchtigkeitsgehalt in der kontrollierten Umgebung korrelierende Feuchte-Messgröße erfasst wird. Hierdurch kann beispielsweise die Konzentration des Dekontaminationsmittels mit dem Feuchtigkeitsgehalt an jedem Referenzpunkt in Beziehung gebracht werden. Alternativ oder zusätzlich kann vorgesehen sein, dass eine mit einer Temperatur in der kontrollierten Umgebung korrelierende Temperatur-Messgröße erfasst wird. Hierdurch kann beispielsweise die Konzentration des Dekontaminationsmittels und/oder die Feuchte mit der Temperatur an jedem Referenzpunkt in Beziehung gebracht werden. Alternativ oder zusätzlich kann vorgesehen sein, dass die zuvor genannten Messgrößen an jedem Messpunkt erfasst werden. Hierdurch kann beispielsweise erreichbar sein, dass die Konzentration des Dekontaminationsmittels und/oder die Feuchte mit der Temperatur an jedem Messpunkt in Beziehung gebracht werden können. Weitere Umweltgrößen wie beispielsweise Luftgeschwindigkeit, Luftdruck können auch erfasst werden.

Bei einer vorteilhaften Ausgestaltung kann vorgesehen sein, dass im Einrichtungsmodus an jedem Referenzpunkt wenigstens ein Messsensor angeordnet wird. Hierdurch kann beispielsweise erreichbar sein, dass der Messsensor an jedem Referenzpunkt eine Messgröße aufzeichnen kann. Alternativ oder zusätzlich kann vorgesehen sein, dass an jedem Messpunkt wenigstens ein Messsensor angeordnet wird. Hierdurch kann beispielsweise erreichbar sein, dass der Messsensor an jedem Messpunkt eine Messgröße aufzeichnen kann. Insbesondere kann vorgesehen sein, dass die Messsensoren des Netzes von Messpunkten vor dem Betriebsmodus aus der kontrollierten Umgebung entfernt werden. Hierdurch kann beispielsweise eine Kalibrierung der Referenzpunkte mit Hilfe der Messpunkte im Einrichtungsmodus erreichbar sein, so dass die Messpunkte in einem, zeitlich späteren, Betriebsmodus nicht benötigt werden, um eine korrekte Aussage über die Konzentration des Dekontaminationsmittels zu ermöglichen. Die im Einrichtungsmodus gemessenen Messgrößen am Referenzpunkt können beispielsweise mit den an den Messpunkten gemessenen Messgrößen ausgewertet werden, um eine Bewertung des Dekontaminationsprozesses im Einrichtungsmodus zu ermöglichen. Im Betriebsmodus kann dann beispielsweise auf die Messpunkte verzichtet werden, so dass im Betriebsmodus eine Bewertung des Dekontaminationsprozesses allein mit den an den Referenzpunkten gemessenen Messgrößen möglich ist.

Bei einer vorteilhaften Ausgestaltung kann vorgesehen sein, dass die Messsensoren an den Messpunkten akkubetrieben sind. Hierdurch kann beispielsweise erreichbar sein, dass die Messpunkte autark, ohne externe Stromversorgung betrieben werden können, wodurch sie vorteilhaft flexibel in der kontrollierten Umgebung angeordnet werden können. Alternativ oder zusätzlich kann vorgesehen sein, dass die Messsensoren zu einer drahtlosen Übermittlung der Messwerte ausgebildet sind. Hierdurch kann beispielsweise erreichbar sein, dass aufgezeichnete Daten kabellos übertragen werden können. Dies hat zudem den Vorteil, dass keine zusätzliche Dekontamination von Kabeln erfolgen muss.

Bei einer vorteilhaften Ausgestaltung kann vorgesehen sein, dass für die Ermittlung des funktionalen Zusammenhangs wenigstens ein Messpunkt, an dem eine Wirksamkeit des eingetragenen Dekontaminationsmittels geringer ist als an dem wenigstens einen Referenzpunkt, automatisiert ermittelt wird. Hierdurch kann beispielsweise die Kalibrierung der Referenzpunkte für den Betriebsmodus verbessert werden. Es sind somit auf einfache Weise Punkte, an denen eine Dekontamination besonders langsam erfolgt, beispielsweise weil sie schwer zugänglich sind, durch Vergleichsmessungen an einfacher zugänglichen Punkten ersetzbar.

Bei einer vorteilhaften Ausgestaltung kann vorgesehen sein, dass die Messwerte jeweils mit einer Wirksamkeitsaussage in einem oder dem Dekontaminationsprozess korrelieren. Hierdurch kann beispielsweise von den Messwerten auf die Wirksamkeit des Dekontaminationsprozesses geschlossen werden.

Bei einer vorteilhaften Ausgestaltung kann vorgesehen sein, dass an jedem Messpunkt mehr als eine physikalische Messgröße erfasst wird. Hierdurch kann beispielsweise erreichbar sein, dass mehrere Messgrößen zur Bewertung des Dekontaminationsprozesses herangezogen werden können und die Bewertung somit verbessert werden kann.

Bei einer vorteilhaften Ausgestaltung kann vorgesehen sein, dass jedem Messpunkt eindeutig eine physikalische Messgröße zugeordnet ist. Hierdurch kann beispielsweise eine physikalische Messgröße an einem Messpunkt erfasst werden. Alternativ oder zusätzlich kann vorgesehen sein, dass das Netz in unterschiedliche Typen von Messpunkten unterteilt ist. Hierdurch kann beispielsweise erreichbar sein, dass das Netz an verschiedenen Messpunkten verschiedenen physikalische Messgrößen messen kann. Es kann auch erreichbar sein, dass an jedem Messpunkt jeweils alle physikalischen Messgrößen gemessen werden können.

Die Erfindung wird nun anhand von Ausführungsbeispielen näher beschrieben, ist jedoch nicht auf die Ausführungsbeispiele beschränkt. Weitere Ausführungsbeispiele ergeben sich durch Kombination der Merkmale einzelner oder mehrerer Ansprüche untereinander und/oder mit einzelnen oder mehreren Merkmalen der Ausführungsbeispiele.

Es zeigen
- Fig. 1: eine kontrollierte Umgebung, in der ein Dekontaminationsmittel auf Objekte einwirkt und
- Fig. 2: eine kontrollierte Umgebung mit einem Netz aus Messpunkten und Referenzpunkten.

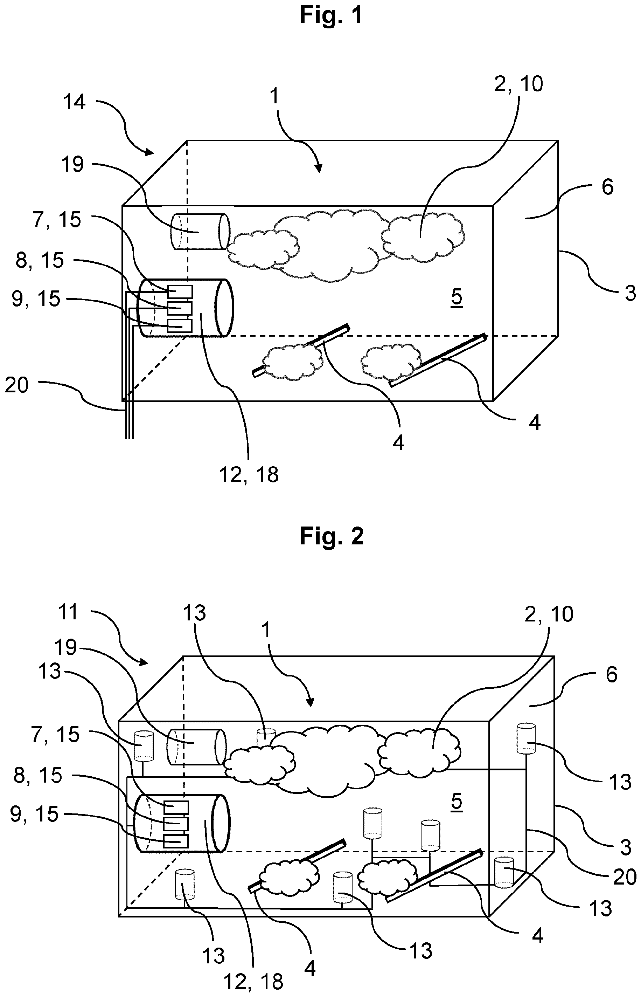

Fig. 1 zeigt ein Verfahren zur Ermittlung einer Wirksamkeitsaussage in einem Dekontaminationsprozess 1. In dem Dekontaminationsprozess 1 wird ein Dekontaminationsmittel 2 in ein kontrollierte Umgebung 3 eingebracht, um dort auf Objekte 4 einzuwirken. Der Dekontaminationsprozess 1 wirkt aber nicht nur auf Objekte in der kontrollierten Umgebung 3, sondern auch auf die im Innenraum 5 vorhandene Luft, sowie die Innenwände 6 der kontrollierten Umgebung 3. Bei dem Verfahren wird eine mit einer Konzentration des Dekontaminationsmittels 2 in der kontrollierten Umgebung 3 korrelierenden Konzentrations-Messgröße 7 erfasst. Auch wird eine mit einem Feuchtigkeitsgehalt in der kontrollierten Umgebung 3 korrelierende Feuchte-Messgröße 8 und eine mit einer Temperatur in der kontrollierten Umgebung 3 korrelierende Temperatur-Messgröße 9 erfasst. Mit den erfassten Messgrößen 7, 8, 9 wird die Wirksamkeitsaussage automatisiert ermittelt.

Für die Ermittlung der Wirksamkeitsaussage wird zusätzlich eine Zeitdauer der Einwirkung des Dekontaminationsmittels 2 verarbeitet. Somit kann eine Einwirkzeit des Dekontaminationsmittels 2 mit den Messgrößen 7, 8, 9 in Beziehung gebracht werden.

Als Dekontaminationsmittel 2 wird gasförmiges Wasserstoffperoxid 10 verwendet.

Die drei Messgrößen 7, 8, 9 werden in der kontrollierten Umgebung 3 an einem gemeinsamen Messort 18 erfasst.

Mit einem Anteil von den Mikroorganismen, die den Dekontaminationsprozess 1 überstehen, wird als Wirksamkeitsaussage eine Kennzahl berechnet, die mit dem Anteil korreliert.

Der funktionale Zusammenhang kann in einem gerätespezifischen Kalibriermodus durch einen Abgleich mit den (nach einer Inkubation erhaltenen) Werten eines Bioindikators und/oder eines (bio)chemischen/enzymatischen Indikators eingelernt werden. Beispielsweise ist dies mit einem neuronalen Netz anhand von Messdaten durchführbar.

Im Betrieb und/oder im Kalibriermodus wird eine relative Sättigung und/oder ein Taupunkt für das Dekontaminationsmittel bestimmt. Hierzu werden Werte gemessen und mit Soll-Werten verglichen, um gewünschte Werte in der Nähe von 100% Sättigung oder bei 100% Sättigung vorzugeben.

Fig. 2 zeigt ein Verfahren zur Bewertung eines Dekontaminationsprozesses 2 der in einer kontrollierten Umgebung 3 ausgeführt wird. In einem (netzwerkspezifischen) Einrichtungsmodus 11 wird ein Referenzpunkt 12 und ein Netz von Messpunkten 13 definiert. Während einer ersten Durchführung des Dekontaminationsprozesses 2 werden Messwerte 16, 17 an dem Referenzpunkt 12 und an den Messpunkten 13 aufgenommen. Zwischen den Messwerten 16 des Referenzpunktes 12 und den Messwerten 17 der Messpunkte 13 wird ein funktioneller Zusammenhang ermittelt.

In einem Betriebsmodus 14, der beispielhaft in Fig. 1 gezeigt ist, werden Messwerte 16 an dem Referenzpunkt 12 aufgenommen, wobei in dem Betriebsmodus 14 auch automatisiert eine auf einen Messpunkt 13 bezogene Information aus den Messwerten 16 mit dem funktionalen Zusammenhang ermittelt wird.

An jedem Referenzpunkt 12 und Messpunkt 13 wird eine mit einer Konzentration des Dekontaminationsmittels 2 in der kontrollierten Umgebung 3 korrelierende Konzentrations-Messgröße 7, sowie eine mit einem Feuchtigkeitsgehalt in der kontrollierten Umgebung 3 korrelierende Feuchte-Messgröße 8 und eine mit einer Temperatur in der kontrollierten Umgebung 3 korrelierende Temperatur-Messgröße 9 erfasst. In einem weiteren Ausführungsbeispiel wird auch eine Luftgeschwindigkeit und ein Luftdruck erfasst.

Im Einrichtungsmodus 11 wird an jedem Referenzpunkt 12 und Messpunkt 13 ein Messsensor 15 angeordnet. Vor einem Wechsel in den Betriebsmodus 14 werden die Messsensoren 15 des Netzes von Messpunkten 13 aus der kontrollierten Umgebung 3 entfernt.

In einem weiteren Ausführungsbeispiel sind die Messsensoren 15 an den Messpunkten 13 akkubetrieben und zu einer drahtlosen Übermittlung der Messwerte 17 ausgebildet.

Fig. 2 zeigt, wie die Messpunkte 13 mit Kabeln 20 verbunden sind. Die in Fig. 1 gezeigten Kabel 20 führen von den Messsensoren 15 aus der kontrollierten Umgebung 3 heraus um außerhalb beispielsweise an einem PC/Laptop/Tablet (nicht gezeigt) ausgewertet zu werden.

Für die Ermittlung des funktionalen Zusammenhangs wird automatisiert ein Messpunkt 13 ermittelt, an dem eine Wirksamkeit des eingetragenen Dekontaminationsmittels 2 geringer ist als an dem Referenzpunkt 12.

Die Messwerte 16, 17 korrelieren jeweils mit einer Wirksamkeitsaussage in dem Dekontaminationsprozess 1.

An jedem Messpunkt 13 wird mehr als eine physikalische Messgröße 7, 8, 9 erfasst.

Jedem Messpunkt 13 ist eindeutig eine physikalische Messgröße 7, 8, 9 zugeordnet. In einem weiteren Ausführungsbeispiel ist das Netz in unterschiedliche Typen von Messpunkten 13 unterteilt.

Die in Fig. 1 und 2 schematisch dargestellte Abgabestelle 19 dient der Verteilung und Abgabe des Dekontaminationsmittels 2 in die kontrollierte Umgebung 3. Hierbei kann es sich beispielsweise um eine Düse handeln.

Es wird ein Verfahren zur Ermittlung einer Wirksamkeitsaussage in einem Dekontaminationsprozess 1 vorgeschlagen, wobei in dem Dekontaminationsprozess 1 ein Dekontaminationsmittel 2 auf wenigstens ein Objekt 4 in einer kontrollierte Umgebung 3 einwirkt, wobei eine mit einer Konzentration des Dekontaminationsmittels 2 in der kontrollierten Umgebung 3 korrelierende Konzentrations-Messgröße 7, eine mit einem Feuchtigkeitsgehalt in der kontrollierten Umgebung korrelierende Feuchte-Messgröße 8 und eine mit einer Temperatur in der kontrollierten Umgebung korrelierende Temperatur-Messgröße 9 erfasst werden so dass mit den erfassten Messgrößen 7, 8, 9 die Wirksamkeitsaussage, vorzugsweise automatisiert, ermittelt wird.

### Bezugszeichenliste

- 1: Dekontaminationsprozess
- 2: Dekontaminationsmittel
- 3: kontrollierte Umgebung
- 4: Objekt
- 5: Innenraum
- 6: Innenwand
- 7: Konzentrations-Messgröße
- 8: Feuchte-Messgröße
- 9: Temperatur-Messgröße
- 10: Wasserstoffperoxid
- 11: Einrichtungsmodus
- 12: Referenzpunkt
- 13: Messpunkt
- 14: Betriebsmodus
- 15: Messsensor
- 16: Messwert des Referenzpunkts
- 17: Messwert des Messpunkts
- 18: Messort
- 19: Abgabestelle
- 20: Kabel

## Patentansprüche

1. Verfahren zur Ermittlung einer Wirksamkeitsaussage in einem Dekontaminationsprozess (1), wobei in dem Dekontaminationsprozess (1) ein Dekontaminationsmittel (2) in eine kontrollierte Umgebung (3) zu einer Einwirkung auf wenigstens ein Objekt (4) eingebracht wird, **dadurch gekennzeichnet, dass** eine mit einer Konzentration des Dekontaminationsmittels (2) in der kontrollierten Umgebung (3) korrelierende Konzentrations-Messgröße (7), eine mit einem Feuchtigkeitsgehalt in der kontrollierten Umgebung korrelierende Feuchte-Messgröße (8) und eine mit einer Temperatur in der kontrollierten Umgebung (3) korrelierende Temperatur-Messgröße (9) erfasst werden und dass mit den erfassten Messgrößen (7, 8, 9) die Wirksamkeitsaussage, vorzugsweise automatisiert, ermittelt wird.

2. Verfahren nach Anspruch 1, **dadurch gekennzeichnet, dass** für die Ermittlung der Wirksamkeitsaussage zusätzlich eine Zeitdauer der Einwirkung des Dekontaminationsmittels (2) in der kontrollierten Umgebung (3) verarbeitet wird, insbesondere wobei die Strömungsgeschwindigkeit einbezogen wird.

3. Verfahren nach einem der vorangehenden Ansprüche, **dadurch gekennzeichnet, dass** als Dekontaminationsmittel (2), vorzugsweise in einer Gasphase aufgenommenes, Wasserstoffperoxid (10) verwendet wird.

4. Verfahren nach einem der vorangehenden Ansprüche, **dadurch gekennzeichnet, dass** wenigstens zwei, insbesondere die drei Messgrößen (7, 8, 9) an einem gemeinsamen Messort (18) in der kontrollierten Umgebung (3) erfasst werden.

5. Verfahren nach einem der vorangehenden Ansprüche, **dadurch gekennzeichnet, dass** als Wirksamkeitsaussage eine Kennzahl, die mit einem Anteil von den den Dekontaminationsprozess (1) überstehenden Mikroorganismen korreliert, berechnet wird.

6. Verfahren nach einem der vorangehenden Ansprüche, **dadurch gekennzeichnet, dass** die Ermittelung der Wirksamkeitsaussage in einem Kalibriermodus anhand von wenigstens einem aufgestellten Bioindikator und/oder wenigstens einem (bio)chemischen/enzymatischen Indikator eingelernt und/oder validiert wird.

7. Verfahren nach einem der vorangehenden Ansprüche, **dadurch gekennzeichnet, dass** eine relative Sättigung und/oder ein Taupunkt für das Dekontaminationsmittel bestimmt, insbesondere vorgegeben und/oder gemessen, wird, insbesondere wobei in der kontrollierten Umgebung eine relative Sättigung von wenigstens 80% oder wenigstens 95%, insbesondere 100%, erreicht wird.

8. Verfahren zur Bewertung eines Dekontaminationsprozesses (1), der in einer kontrollierten Umgebung (3) ausgeführt wird, insbesondere unter Verwendung eines Verfahrens nach einem der vorangehenden Ansprüche, **dadurch gekennzeichnet, dass** in einem Einrichtungsmodus (11) wenigstens ein Referenzpunkt (12) und ein Netz von mehreren Messpunkten (13) in der kontrollierten Umgebung (3) definiert wird, wobei während einer ersten Durchführung des Dekontaminationsprozesses (1) Messwerte (16, 17) an dem wenigstens einen Referenzpunkt (12) und an den Messpunkten (13) aufgenommen werden und wobei ein funktioneller Zusammenhang zwischen den Messwerten (16) des wenigstens einen Referenzpunktes (12) und den Messwerten (17) der Messpunkte (13) ermittelt wird, und dass in einem Betriebsmodus(14) wenigstens ein Messwert (16) des wenigstens einen Referenzpunktes (12) aufgenommen wird und dass in dem Betriebsmodus (14) aus dem wenigstens einen aufgenommenen Messwert (16) mit dem funktionalen Zusammenhang wenigstens eine auf wenigstens einen Messpunkt (13) des Netzes bezogene Information automatisiert ermittelt wird.

9. Verfahren nach dem vorangehenden Anspruch, **dadurch gekennzeichnet, dass** an jedem Referenzpunkt (12) und/oder Messpunkt (13) wenigstens eine mit einer Konzentration des Dekontaminationsmittels (2) in der kontrollierten Umgebung (3) korrelierende Konzentrations-Messgröße (7), eine mit einem Feuchtigkeitsgehalt in der kontrollierten Umgebung (3) korrelierende Feuchte-Messgröße (8) und/oder eine mit einer Temperatur in der kontrollierten Umgebung (3) korrelierende Temperatur-Messgröße (9) erfasst wird/werden.

10. Verfahren nach einem der Ansprüche 8 oder 9, **dadurch gekennzeichnet, dass** im Einrichtungsmodus (11) an jedem Referenzpunkt (12) und/oder Messpunkt (13) wenigstens ein Messsensor (15) angeordnet wird, insbesondere wobei die Messsensoren (15) des Netzes von Messpunkten (13) vor dem Betriebsmodus (14) aus der kontrollierten Umgebung (4) entfernt werden.

11. Verfahren nach einem der Ansprüche 8 bis 10, **dadurch gekennzeichnet, dass** die Messsensoren (15) an den Messpunkten (13) akkubetrieben und/oder zu einer drahtlosen Übermittlung der Messwerte (17) ausgebildet sind.

12. Verfahren nach einem der Ansprüche 8 bis 11, **dadurch gekennzeichnet, dass** für die Ermittlung des funktionalen Zusammenhangs wenigstens ein Messpunkt (13), an dem eine Wirksamkeit des eingetragenen Dekontaminationsmittels (2) geringer ist als an dem wenigstens einen Referenzpunkt (12), automatisiert ermittelt wird.

13. Verfahren nach einem der Ansprüche 8 bis 12, **dadurch gekennzeichnet, dass** die Messwerte (16, 17) jeweils mit einer Wirksamkeitsaussage in einem oder dem Dekontaminationsprozess (1) korrelieren.

14. Verfahren nach einem der Ansprüche 8 bis 13, **dadurch gekennzeichnet, dass** an jedem Messpunkt (13) mehr als eine physikalische Messgröße (7, 8, 9) erfasst wird.

15. Verfahren nach einem der Ansprüche 8 bis 14, **dadurch gekennzeichnet, dass** jedem Messpunkt (13) eindeutig eine physikalische Messgröße (7, 8, 9) zugeordnet ist und/oder dass das Netz in unterschiedliche Typen von Messpunkten (13) unterteilt ist.
